# EUROPEAN PATENT SPECIFICATION

(11) **EP 1 523 273 B1**
(45) Date of publication and mention of the grant of the patent: **18.03.2009**
(21) Application number: 02770331.3
(22) Date of filing: 15.07.2002
(51) Int. Cl.: A61B 8/00, A61B 8/12

(54) **A MECHANISM AND SYSTEM FOR 3-DIMENTIONAL SCANNING OF AN ULTRASOUND BEAM**
SYSTEM UND MECHANISMUS FÜR DREIDIMENSIONALE ABTASTUNG EINES ULTRASCHALLBÜNDELS
MECANISME ET SYSTEME PERMETTANT LE BALAYAGE D'UN FAISCEAU ULTRASONORE DANS UN ESPACE 3D

(43) Date of publication of application: 20.04.2005
(73) Proprietor: TeraRecon, Inc., San Mateo, CA 94402 (US)
(72) Inventor: ANGELSEN, Bjorn, A., J., N-7051 Trondheim (NO); JOHANSEN, Tonni, F., N-7026 Trondheim (NO); KJODE, Stig, N-6723 Svelgen (NO)
(74) Representative: Hitchcock, Esmond Antony
(86) International application number: PCT/NO2002/000264
(87) International publication number: WO 2004/006773

(56) References cited:
- EP-A- 1 103 222
- US-A- 4 271 706
- US-A- 4 398 425
- US-A- 4 399 703
- US-A- 4 579 122
- US-A- 4 841 979
- US-A- 5 094 243
- US-A- 6 120 452
- Diagnostic Ultrasound, F. W. Kremkau, pages 92-105, 1993

## Description

The present invention is directed to technology for scanning an ultrasound beam in a free direction within a region of 3D space. The technology has particular applications within 3D medical ultrasound imaging, but also has applications in other areas of ultrasound imaging or other areas where mechanical movement of an object in 3D space is required.

Three-dimensional (3D) ultrasound imaging of biological structures can be done by scanning a pulsed ultrasound beam in a 3D manner, and recording the back-scattered ultrasound signal in each beam direction. The principle was described already in the 50'ies, and several instruments have been build that applies the method.

With 3D imaging, it is important that the ultrasound beam is maximally focused, symmetrically in all directions around the beam axis, as one wants to observe the object from any direction (perspective), and small objects can be interrogated with a variety of beam directions. Such symmetric focusing can be obtained by subdividing the elements of a linear transducer array in the elevation direction, i.e. the direction normal to the two-dimensional (2D) scan plane. The 2D scan plane is often referred to as the azimuth scan plane, while the direction normal to the 2D scan plane is referred to as the elevation direction. Linear arrays with subdivision of the elements in the elevation direction are referred to as 1.5D or 1.75D arrays, depending on whether the elements can be symmetrically or individually steered around the azimuth mid plane. A problem with the subdivision of the elements is that the size of the elements are reduced, increasing the element impedance and hence also noise. The subdivision also gives a large total number of elements, leading to a large number of wires between the array elements and the electronic beam former. This increases cable losses and hence sensitivity of the array.

Symmetric focusing of the beam can be obtained with much fewer and larger elements with an array of concentric annular elements, the so-called annular array. The larger elements provide lower element impedances and hence less noise and cable losses. Another advantage with the annular array, is that it is covered in a dome, so that the array itself is not pushed against the body or other objects as the linear arrays typically are done. This allows the use of materials with lower characteristic impedance for mechanical backing support with the annular array compared to the linear arrays, giving less acoustic backing losses and hence better sensitivity with the annular array.

Hence the annular array provides better sensitivity for symmetrical focusing of the beam around the beam axis than the linear arrays, due to the large elements and low impedance backing material. The increased sensitivity in turn allows the use of higher ultrasound frequencies and hence better resolution for a given depth, than the linear arrays. The low number of elements also reduces the number of connecting cables and hence gives a lower manufacturing cost of the array.

EP 1 103 222 discloses an ultrasonic probe comprising a continuously rotating cylinder carrying a piezoelectric element. The rotational axis of the cylinder can be moved.

The present invention there is an ultrasound transducer probe for scanning the direction and focus of an ultrasound beam in 3D space, comprising:
a first electric motor comprising a rotor and a stator, where the stator of the first electric motor is mounted in a fork and the rotor of the first electric motor is attached to a first shaft, enabling the first shaft to rotate in the bearing mounted in the fork;
an annular ultrasound transducer array attached to the rotor; a second electric motor arranged to move the fork so that the first shaft is moved, so that through electric drive signals on the motors the transducer array is capable of being directed in any direction within a region of 3D space;
the probe
further arranged to measure the angular position of the first shaft and the position of the fork and to feed position signals to a feed-back controller arranged to provide drive signals for the electric motors, so that the direction of the ultrasound beam is steered so that the measured positions of the first shaft and the fork follow close to a reference signal.

A problem with the annular array for 3D beam scanning, is that scanning of the beam direction requires mechanical movement of the array. 3D scanning of the beam with small size of the whole scanning mechanism is then difficult, and the present invention provides a solution to this problem. With a particular embodiment of the invention, the annular array beam can be scanned in a 3D sector with more than 200 deg opening angle in both the elevation and the azimuth directions, with a dimension of the scan-head only slightly larger than the active radiation aperture of the array. The narrow head and wide scanning angle provide a probe that is small and easy to handle, and has special advantages for endoluminal and surgical imaging, where the probe is placed in narrow channels with limited possibilities to direction steer the probe, like for example with transvaginal, transrectal, transesophageal, transgastric, and transintestinal imaging, and imaging from narrow surgical wounds.

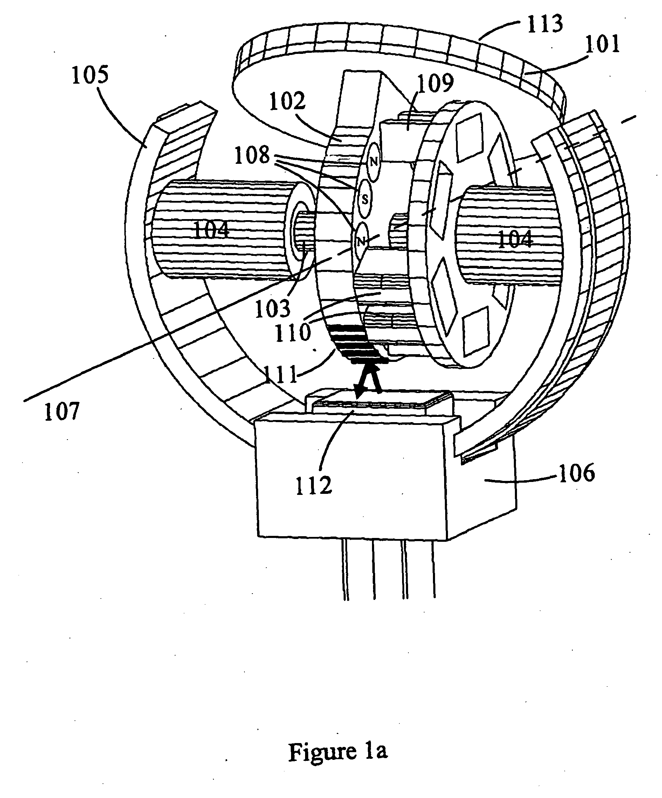

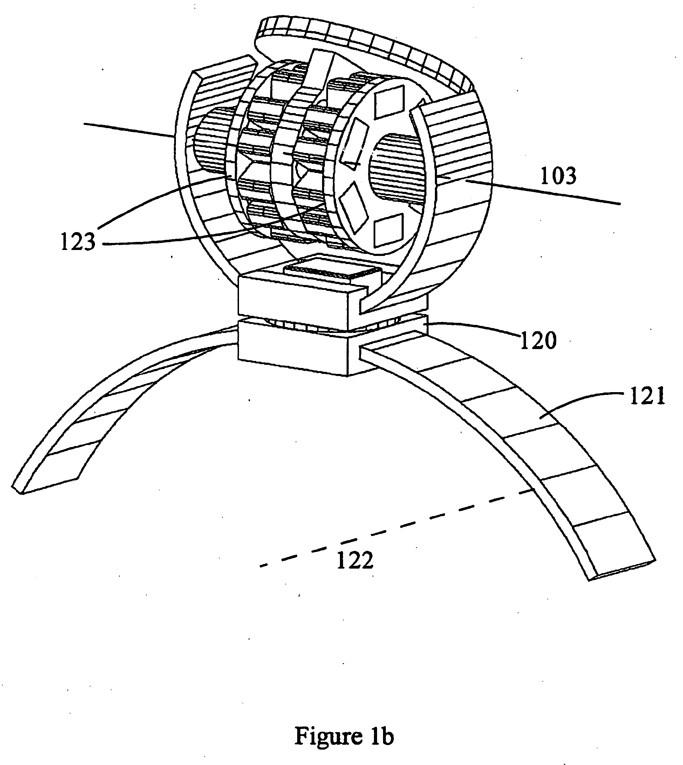

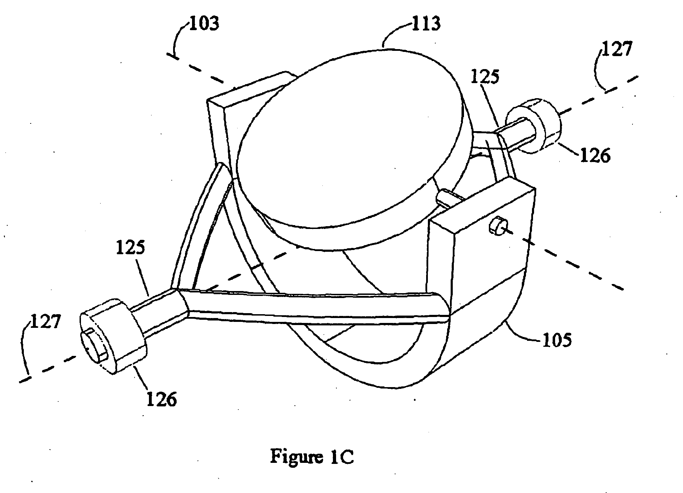

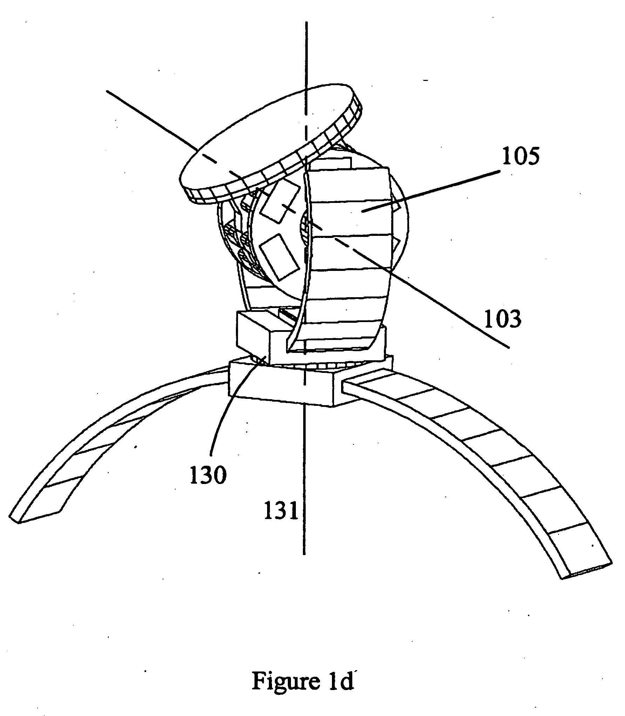

Figures 1a, 1b and 1d show particular embodiments according to the invention that provide rotation of an annular transducer array around an azimuth axis defined by a 1^{st} shaft that rotates in bearings mounted in a fork that can be moved, where Figure la shows sliding of the fork in a sliding system that provides rotation of the array around an elevation axis normal and close to the azimuth axis, Figure 1b shows sliding of the fork in a sliding system that provides rotation of the array around an elevation axis normal to the azimuth axis and at a distance from the azimuth axis. Figure 1c shows rotation of the fork around a 2^{nd} shaft that provides rotation of the array around an elevation axis, and Figure 1d shows combined rotation of fork around a roll axis. Fig. 1c is not part of the present invention.

Figure 2 shows an example of a control system for driving the rotation of the transducer around the azimuth axis to follow an azimuth reference signal.

Figure 3 shows a system with flexible bands thread around a pully wheel attached to an electric motor for moving the fork in a sliding system.

A particular embodiment of the invention will be described with reference to the drawings. In **Figure 1a** 101 shows an annular array that is attached to the rotor 102 of an electric motor that is attached to a shaft 103 that is free to move in bearings 104 mounted in a fork 105. Rotating the array around the shaft 103 scans the ultrasound beam in an angular direction referred to as the azimuth direction, within a 2D plane referred to as the azimuth plane. The fork 105 is mounted in a sliding system 106 which allows movement of the shaft. In this particular embodiment, the fork has a circular shape so that sliding of the fork through the sliding system produces a rotation of the shaft 103 around an axis 107, normal to the shaft, which allows angular scanning of the beam in what is referred to as the elevation direction. Moreover, this particular embodiment produces a rotation of the shaft around an axis on the same side of the sliding system as the shaft itself, and the axis 107 goes through the center of the shaft 103. This embodiment allows for a compact size of the mechanism in relation to the size of radiating acoustic surface aperture 113 of the transducer array 101.

**Figure 1b** shows a modification of the embodiment where the sliding system 120 slides along a fork 121, arranged so that the shaft 103 rotates around an axis 122 on the side of the sliding system opposite to the shaft axis 103. An advantage of this sliding system is that the width of the beam scanning area becomes wider at the probe surface, than for the scanning system in Figure 1a. The scanning system in Figure 1a, however, produces a small outer dimension of the probe.

The rotor 102 of Figure 1a contains a set of permanent magnets distributed around the rotor in a sequence with opposite polarities, where in the drawing 3 magnets 108 are visible. Rotation of the rotor 102 causes the magnets to slide between the poles of a set of electromagnets with iron cores 109 with surrounding coils 110. In Figure 1a, some of the magnets are removed for clarity, the cores 109 are shown without coils for some magnets, while the coils 110 around the cores are shown for other magnets. In Figure 1b an embodiment of an entire electromagnet system 123 is shown. By controlling the current in the coils according to standard methods for those skilled in the art, a torque is developed on the rotor that causes the transducer array to rotate around the shaft axis 103.

For this particular embodiment, an optical reflectance grating 111 in Figure 1a is mounted around the rotor periphery. The grating is illuminated with a light emitting diode contained in the unit 112. The light reflected from the grating is picked up with two light detection diodes in the unit 112 that are offset a distance. By monitoring the signals from the two detector diodes one can both observe the direction of movement of the rotor, and its rotation angle measured in steps of 1/4 of the angle between the reflection stripes of the position grating 111, according to well known principles.

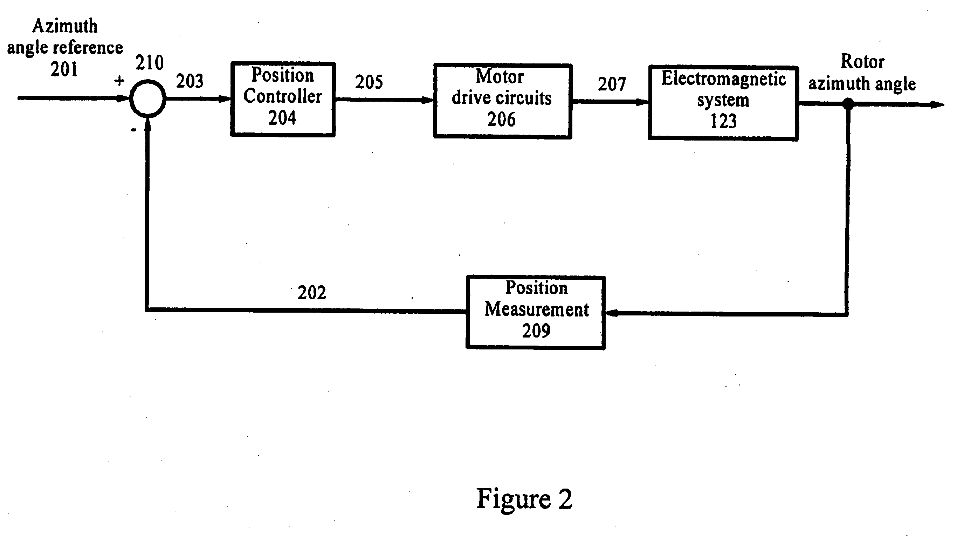

The system hence provides both a motor to produce a torque on the shaft 103 and a position system to measure the direction and angular rotation of the transducer array around the shaft axis. The motor and the position measurement system can hence be connected in a feed-back control system for rotation of the transducer array around the shaft 103 under feed-back control. This allows precise direction steering of the array around the shaft axis, for example with an electronic control system as shown in **Figure 2****.**

In this Figure, the azimuth angle of the transducer array is measured in a unit 209, which includes 111 and 112 of Figure 1a, producing a measured azimuth angle signal 202. The measured azimuth angle signal 202 is compared with an azimuth angle reference signal 201 in the unit 210 that produces the angular position error 203 as the difference between the reference and the measured position. The position error signal is fed to a controller unit 204 that provides inputs 205 to the motor drive unit 206 that provides a set of electric drive signals 207 for the motor electromagnetic system 123 of Figure 1b. The control system hence drives the rotor azimuth angle to follow the azimuth angle reference.

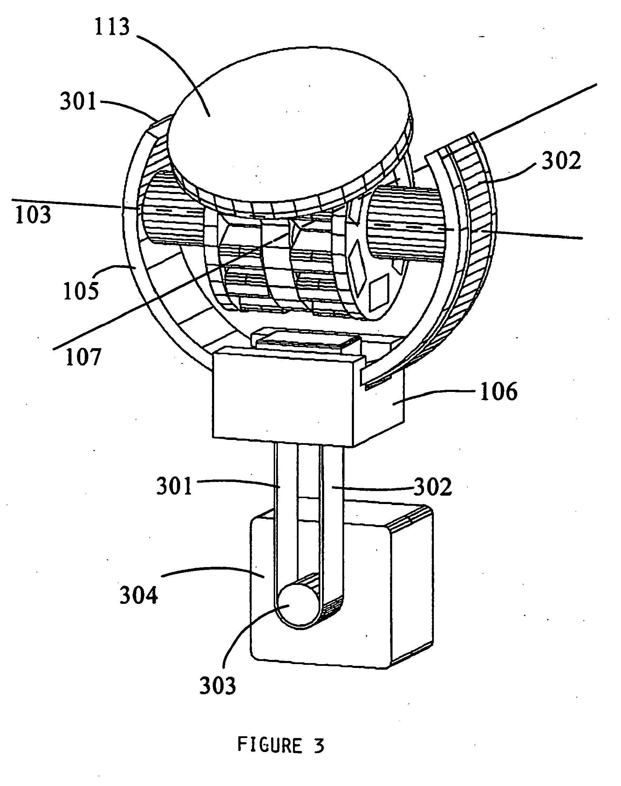

To scan the beam in the elevation direction in Figure 1a, the fork is moved in the sliding system 106. A system to control such sliding according to the embodiment, is shown in **Figure 3****.** Two flexible bands 301 and 302 are attached to the fork 105, so that when one of the bands are pulled, the fork slides in the sliding system 106. This sliding produces a movement of the azimuth rotation axis of the shaft 103, where in this particular embodiment the shaft rotates around an elevation axis 107 normal to the shaft axis. The combined rotation around the azimuth shaft axis 103 and the elevation fork axis 107, produces a 3D sector scanning of the beam in both the azimuth and the elevation directions. In this embodiment, the two flexible bands 301 and 302 are connected together in a loop around a pully wheel 303 connected to a rotating electrical motor 304. Angle controlled rotation of the motor 304, for example by a control system similar to that in Figure 2, causes a controlled rotation of the transducer around the elevation axis 107_{.}

Movement of the azimuth rotation axis 103 as illustrated in **Figure 1c****,** where the fork 105 is connected to a 2^{nd} shaft 125 that can rotate in the bearing system 126 to rotate the array 113 around both the elevation axis 127 and the azimuth axis 103, is not part of the present invention.

Movement of the azimuth rotation axis 103 is illustrated in **Figure 1d****,** where the fork 105 is mounted in a shaft-bearing system 130 that allows the fork and hence the azimuth axis 103 to rotate around the roll axis 131, normal to the azimuth axis. Combined rotation around the azimuth axis 103 and the roll axis 131 produces a 3D scanning of the beam direction. Rotation around the roll axis 131 can be obtained by an electric motor with direct coupling, coupling through a gear or through a set of pullies.

## Claims

1. An ultrasound transducer probe for scanning the direction and focus of an ultrasound beam in 3D space, comprising:
a first electric motor comprising a rotor and a stator, where the stator of the first electric motor is mounted in a fork (105) and the rotor of the first electric motor is attached to a first shaft (103), enabling the first shaft (103) to rotate in the bearing mounted in the fork (105);
an annular ultrasound transducer array (101, 113) attached to the rotor;
a second electric motor arranged to move the fork (105) so that the first shaft (103) is moved, so that through electric drive signals on the motors the transducer array (101, 113) is capable of being directed in any direction within a region of 3D space;
the probe
further arranged to measure the angular position of the first shaft (103) and the position of the fork (105) and to feed position signals to a feed-back controller arranged to provide drive signals for the electric motors, so that the direction of the ultrasound beam is steered so that the measured positions of the first shaft (103) and the fork (105) follow close to a reference signal.

2. An ultrasound transducer probe according to claim 1, where the fork (105) is moved through sliding in a sliding system (106).

3. An ultrasound transducer probe according to claim 2, where the fork (105) has a circular shape and the sliding system (106) is mounted so relative to the fork (105) that the sliding provides a rotation of the first shaft around (103) an axis (107; 122) normal to the first shaft (103).

4. An ultrasound transducer probe according to claim 2, where the sliding system is mounted so relative to the fork (105) that the sliding provides a lateral displacement of the first shaft (103) in the plane of the first shaft (103).

5. An ultrasound transducer probe according to claim 2, where the sliding system (106) is mounted so relative to the fork (105) that the sliding provides a rotation of the first shaft (103) around an axis (107) on the first shaft (103) side of the sliding system (106), normal to the first shaft (103).

6. An ultrasound transducer probe according to claim 1, where the fork (105) is attached to at least one flexible band (301, 302), and the movement of the fork (105) is obtained by pulling the flexible band (301, 302).

7. An ultrasound transducer probe according to claim 6, where the fork (105) is attached to a flexible band (301, 302)on one side, and a spring system on the other side so that bi-directional movement of the fork (105) is obtained by combined action of the spring system and pulling of the flexible band (301, 302),
where pulling of the flexible band (301, 302) moves the fork (105) in one direction and the spring pulls the fork (105) in the opposite direction when the pull in the flexible band (301, 302) is released.

8. An ultrasound transducer probe according to claim 6, where the fork (105) on each side is attached to separate flexible band (301, 302), and bi-directional movement of the fork (105) is obtained by selectively pulling one of the bands (301, 302).

9. An ultrasound transducer probe according to claim 6, where the pulling of the flexible band(s) (301, 302) is provided for by the second electric motor.

10. An ultrasound transducer probe according to claim 2, where a second shaft-bearing (130) system is mounted to a sliding system that allows movement of position and direction of the second shaft-bearing system (130), a third electric motor being connected to the sliding system to move the position and direction of the second shaft-bearing system (130).

11. An ultrasound transducer probe according to claim 3, wherein the axis (107; 122) normal to the first shaft (103) is located on the same side of the sliding system (106) as the first shaft (103) or on the side of the sliding system opposite to the first shaft (103).

## Patentansprüche

1. Ultraschallwandlersonde zum Abtasten der Richtung und des Fokus eines Ultraschallbündels im 3D-Raum, umfassend:
einen ersten Elektromotor, der einen Rotor und einen Stator umfasst, wo der Stator des ersten Elektromotors auf eine Gabel (105) montiert ist und der Rotor des ersten Elektromotors an einer ersten Welle (103) befestigt ist, wodurch der ersten Welle (103) ermöglicht wird, sich in dem in die Gabel (105) montierten Lager zu drehen;
ein ringförmiges Ultraschallwandlerarray (101, 113), das am Rotor befestigt ist;
einen zweiten Elektromotor, der zum Bewegen der Gabel (105) angeordnet ist, sodass die erste Welle (103) bewegt wird, sodass das Wandlerarray (101, 113) durch elektrische Ansteuersignale an die Motoren in jede Richtung innerhalb eines Bereichs des 3D-Raums gelenkt werden kann;
wobei die Sonde außerdem angeordnet ist, um die Winkelposition der ersten Welle (103) und die Position der Gabel (105) zu messen und Positionssignale an einen Feedback-Controller anzulegen, der dazu angeordnet ist, Ansteuersignale für die Elektromotoren bereitzustellen, sodass die Richtung des Ultraschallbündels so gesteuert wird, dass die gemessenen Positionen der ersten Welle (103) und der Gabel (105) einem Referenzsignal gut folgen.

2. Ultraschallwandlersonde nach Anspruch 1, wo die Gabel (105) durch Gleiten in einem Gleitsystem (106) bewegt wird.

3. Ultraschallwandlersonde nach Anspruch 2, wo die Gabel (105) kreisförmig ist und das Gleitsystem (106) relativ zur Gabel (105) so montiert ist, dass das Gleiten eine Drehung der ersten Welle (103) um eine Achse (107; 122) bereitstellt, die senkrecht zur ersten Welle (103) ist.

4. Ultraschallwandlersonde nach Anspruch 2, wo das Gleitsystem relativ zur Gabel (105) so montiert ist, dass das Gleiten einen lateralen Versatz der ersten Welle (103) in der Ebene der ersten Welle (103) erzeugt.

5. Ultraschallwandlersonde nach Anspruch 2, wo das Gleitsystem (106) relativ zur Gabel (105) so montiert ist, dass das Gleiten eine Drehung der ersten Welle (103) um eine Achse (107) auf der Seite der ersten Welle (103) des Gleitsystems (106) erzeugt, die senkrecht zur ersten Welle (103) ist.

6. Ultraschallwandlersonde nach Anspruch 1, wo die Gabel (105) an mindestens ein flexibles Band (301, 302) befestigt ist und die Bewegung der Gabel (105) durch Ziehen des flexiblen Bands (301, 302) erzeugt wird.

7. Ultraschallwandlersonde nach Anspruch 6, wo die Gabel (105) auf einer Seite an ein flexibles Band (301, 302) und auf der anderen Seite an ein Federsystem befestigt ist, sodass bidirektionale Bewegung der Gabel (105) durch kombinierte Wirkung des Federsystems und des Ziehens des flexiblen Bands (301, 302) erzeugt wird,
wo das Ziehen des flexiblen Bands (301, 302) die Gabel (105) in eine Richtung bewegt und die Feder die Gabel (105) in die entgegengesetzt Richtung zieht, wenn der Zug im flexiblen Band (301, 302) entspannt wird.

8. Ultraschallwandlersonde nach Anspruch 6, wo die Gabel (105) auf jeder Seite an ein separates flexibles Band (301, 302) befestigt ist und die bidirektionale Bewegung der Gabel (105) durch selektives Ziehen eines der Bänder (301, 302) erzeugt wird.

9. Ultraschallwandlersonde nach Anspruch 6, wo das Ziehen des mindestens einen flexiblen Bands (301, 302) durch den zweiten Elektromotor erzeugt wird.

10. Ultraschallwandlersonde nach Anspruch 2, wo ein zweites Wellenlagersystem (130) an ein Gleitsystem montiert ist, das Bewegung von Position und Richtung des zweiten Wellenlagersystems (130) ermöglicht, wobei ein dritter Elektromotor an das Gleitsystem angeschlossen ist, um die Position und Richtung des zweiten Wellenlagersystems (130) zu bewegen.

11. Ultraschallwandlersonde nach Anspruch 3, worin die zur ersten Welle (103) senkrechte Achse (107; 122) auf derselben Seite des Gleitsystems (106) wie die erste Welle (103) oder auf der der ersten Welle (103) entgegengesetzten Seite des Gleitsystems angeordnet ist.

## Revendications

1. Sonde de transducteur ultrasonore pour balayer la direction et le foyer d'un faisceau ultrasonore dans un espace 3D, comportant:
un premier moteur électrique comprenant un rotor et un stator, dans lequel le stator du premier moteur électrique est monté dans une fourche (105) et le rotor du premier moteur électrique est fixé à un premier arbre (103), permettant au premier arbre (103) de tourner dans le palier monté dans la fourche (105) ;
un réseau de transducteurs ultrasonores en anneau (101, 113) fixé au rotor ;
un deuxième moteur électrique agencé pour déplacer la fourche (105) afin que le premier arbre (103) soit déplacé, de sorte que, via des signaux d'attaque électriques sur les moteurs, le réseau de transducteurs (101, 113) est apte à être dirigé dans une quelconque direction au sein d'une région de l'espace 3D;
la sonde est en outre agencée pour mesurer la position angulaire du premier arbre (103) et la position de la fourche (105) et pour introduire des signaux de position dans un contrôleur de rétroaction agencé pour délivrer des signaux d'attaque pour les moteurs électriques, de sorte que la direction du faisceau ultrasonore est dirigée de sorte que les positions mesurées du premier arbre (103) et de la fourche (105) suivent de près un signal de référence.

2. Sonde de transducteur ultrasonore selon la revendication 1, dans laquelle la fourche (105) est déplacée par glissement dans un système de glissement (106).

3. Sonde de transducteur ultrasonore selon la revendication 2, dans laquelle la fourche (105) a une forme circulaire et le système de glissement (106) est monté d'une telle manière, relativement à la fourche, (105) que le glissement délivre une rotation du premier arbre (103) autour d'un axe (107 ; 122) perpendiculaire au premier arbre (103).

4. Sonde de transducteur ultrasonore selon la revendication 2, dans laquelle le système de glissement est monté d'une telle manière, relativement à la fourche (105), que le glissement délivre un déplacement latéral du premier arbre (103) dans le plan du premier arbre (103).

5. Sonde de transducteur ultrasonore selon la revendication 2, dans laquelle le système de glissement (106) est monté d'une telle manière, relativement à la fourche (105), que le glissement délivre une rotation du premier arbre (103) autour d'un axe (107) sur le côté du premier arbre (103) du système de glissement (106), perpendiculaire au premier arbre (103).

6. Sonde de transducteur ultrasonore selon la revendication 1, dans laquelle la fourche (105) est fixée à au moins une bande flexible (301, 302), et le mouvement de la fourche (105) est obtenu en tirant sur la bande flexible (301, 302).

7. Sonde de transducteur ultrasonore selon la revendication 6, dans laquelle la fourche (105) est fixée à une bande flexible (301, 302) sur un côté, et à un système à ressort sur l'autre côté, de sorte qu'un mouvement bidirectionnel de la fourche (105) est obtenu par une action combinée du système à ressort et en tirant sur la bande flexible (301, 302),
où la traction de la bande flexible (301, 302) déplace la fourche (105) dans une direction et le ressort tire la fourche (105) dans la direction opposée, lorsque la traction sur la bande flexible (301, 302) est relâchée.

8. Sonde de transducteur ultrasonore selon la revendication 6, dans laquelle la fourche (105) est fixée de chaque côté à une bande flexible séparée (301, 302), et un mouvement bidirectionnel de la fourche (105) est obtenu en tirant de manière sélective sur l'une des bandes (301, 302).

9. Sonde de transducteur ultrasonore selon la revendication 6, dans laquelle la traction de la ou des bandes flexibles (301, 302) est délivrée par le deuxième moteur électrique.

10. Sonde de transducteur ultrasonore selon la revendication 2, dans laquelle un second système de palier (130) est monté dans un système de glissement qui permet un mouvement de position et de direction du second système de palier (130), un troisième moteur électrique étant connecté au système de glissement pour déplacer la position et la direction du second système de palier (130).

11. Sonde de transducteur ultrasonore selon la revendication 3, dans laquelle l'axe (107 ; 122) perpendiculaire au premier arbre (103) est situé sur le même côté du système de glissement (106) que le premier arbre (103) ou sur le côté du système de glissement opposé au premier arbre (103).
